# EUROPÄISCHE PATENTANMELDUNG

(11) **EP 2 840 869 A2**
(43) Veröffentlichungstag der Anmeldung: **25.02.2015**
(21) Anmeldenummer: 14175496.0
(22) Anmeldetag: 03.07.2014
(51) Int. Cl.: H05B 37/02

(54) **Steuervorrichtung für ein Leuchtmittel sowie Steuerverfahren für wenigstens ein Leuchtmittel**

(30) Priorität: 08.08.2013 DE 102013108554
(71) Anmelder: Insta Elektro GmbH, 58509 Lüdenscheid (DE)
(72) Erfinder: Grosch, Volker, 45549 Sprockhövel (DE); Melnik, Andrew, 44225 Dortmund (DE)

(57) **Zusammenfassung**

Die Erfindung betrifft eine Steuervorrichtung 10 für wenigstens ein Leuchtmittel 5. Um eine verbesserte Steuerung von Leuchtmitteln innerhalb eines Beleuchtungssystems zu ermöglichen ist erfindungsgemäß vorgesehen, dass die Steuervorrichtung
- wenigstens eine erste Schnittstelle 11 zum Anschluss an einen Daten-Bus 3 einer Beleuchtungssteuerung 2,
- wenigstens eine zweite Schnittstelle 12 zum Anschluss an wenigstens ein elektronisches Vorschaltgerät 4 des wenigstens einen Leuchtmittels 5 sowie
- einen Konverter 13 der dazu eingerichtet ist, über die erste Schnittstelle 11 erste Steuerungsdaten zu empfangen, die für das wenigstens eine Vorschaltgerät 4 bestimmt sind, und aus den ersten Steuerungsdaten gemäß einer Parametrierung zweite Steuerungsdaten zu erzeugen, die sich von den ersten Steuerungsdaten unterscheiden, wobei die Parametrierung gegenüber einem Reset seitens der Beleuchtungssteuerung 2 geschützt ist,
umfasst, wobei die Steuervorrichtung 10 dazu eingerichtet ist, die zweiten Steuerungsdaten über die zweite Schnittstelle 12 an das wenigstens eine Vorschaltgerät 4 zu senden.

Beschrieben ist des Weiteren ein Steuerungsverfahren für wenigstens ein Leuchtmittel.

## Beschreibung

Die Erfindung betrifft eine Steuervorrichtung sowie ein Steuerverfahren für wenigstens ein Leuchtmittel.

In modernen Gebäudeinstallationssystemen kommen nahezu ausschließlich Leuchten zu Einsatz, die mit einem elektronischen Vorschaltgerät (EVG) versehen sind, das die Stromaufnahme des eigentlichen Leuchtmittels (z.B. einer oder mehrerer LEDs) regelt. Typischerweise sind die Leuchten an ein Bus-System angeschlossen, über welches die EVGs angesteuert werden. Für derartige Bus-Systeme hat sich heute weitgehend der DALI-Standard (Digital Addressable Lighting Interface) durchgesetzt, daneben sind z.B. auch der digitale DSI-Standard sowie der analoge 1-10V-Standard gebräuchlich.

Über eine zentrale Beleuchtungssteuerung, die an den Bus angeschlossen ist, können beispielsweise bei DALI-Systemen Betriebsparameter der einzelnen Vorschaltgeräte eingestellt werden. Eine individuelle Parametrierung der elektrischen Vorschaltgeräte bringt allerdings Probleme mit sich. So nimmt die Effizienz von LED-Leuchten mit der Zeit ab, d.h. bei gleichem Betriebsstrom werden diese mit zunehmender Betriebsdauer dunkler. Darüber hinaus kann es sein, dass Leuchten unterschiedlicher Hersteller bzw. unterschiedlicher Produktgenerationen unterschiedlich effizient arbeiten und somit bei gleichem Betriebsstrom eine unterschiedliche Helligkeit liefern.

Um dieses zu kompensieren, kann jedes elektronische Vorschaltgerät entsprechend parametriert werden, was aber zum einen aufwändig ist. Zum anderen gehen diese im Vorschaltgerät abgelegten Parameter verloren, wenn seitens der Beleuchtungssteuerung ein Reset ausgelöst wird. Durch diesen werden sämtliche diesbezüglichen Eintragungen auf die Ursprungswerte zurückgesetzt. Ein solcher Reset betrifft auch etwaige in der Beleuchtungssteuerung zentral hinterlegten Parameter.

Vor diesem Hintergrund besteht die der Erfindung zugrunde liegende Aufgabe darin, eine verbesserte Steuerung von Leuchtmitteln innerhalb eines Beleuchtungssystems zu ermöglichen.

Die Aufgabe wird erfindungsgemäß durch eine Steuervorrichtung mit den Merkmalen des Anspruchs 1 sowie durch ein Steuerverfahren mit den Merkmalen des Anspruchs 13 gelöst.

Durch die Erfindung wird eine Steuervorrichtung für wenigstens ein Leuchtmittel zur Verfügung gestellt. Als Leuchtmittel kommen hierbei beispielsweise Leuchtstofflampen, Gasentladungslampen sowie verschiedene Typen von Leuchtdioden (LEDs) infrage. Die Steuervorrichtung kann insbesondere auch für eine Mehrzahl von Leuchtmitteln vorgesehen sein.

Die Steuervorrichtung umfasst wenigstens eine erste Schnittstelle zum Anschluss an einen Daten-Bus einer Beleuchtungssteuerung. Der Daten-Bus ist hierbei dazu ausgelegt, dass eine Beleuchtungssteuerung hierüber bestimmte Steuerungsdaten an ein oder mehrere Leuchtmittel senden kann, um deren Betrieb zu steuern, und ggf. auch Daten von den Leuchtmitteln empfangen kann. Die Beleuchtungssteuerung sowie der Daten-Bus können hierbei insbesondere nach dem DALI-Standard, ggf. aber auch nach dem DSI-, DMX- oder 1-10V-Standard ausgestaltet sein. Im letztgenannten Fall bezieht sich der Begriff "Steuerungsdaten" auf das analoge Steuerungssignal.

Weiterhin umfasst die Steuervorrichtung eine zweite Schnittstelle zum Anschluss an wenigstens ein elektronisches Vorschaltgerät des wenigstens einen Leuchtmittels. Der Begriff "elektronisches Vorschaltgerät" ist hierbei breit auszulegen und umfasst sämtliche Geräte, die dazu dienen, den Strom durch das Leuchtmittel zu regulieren oder zu begrenzen. Hierunter fallen insbesondere Treiber für Leuchtdioden, aber auch Vorschaltgeräte für Leuchtstofflampen etc. Namentlich kann es sich um einen digital dimmbares Vorschaltgerät handeln. Die zweite Schnittstelle ist somit dafür ausgelegt, direkt oder indirekt (durch eine dazwischen liegende Leitung) an eine Eingangsschnittstelle des elektronischen Vorschaltgeräts angeschlossen zu werden, über welche dieses üblicherweise Steuerungsdaten unmittelbar aus dem Daten-Bus empfängt.

Schließlich umfasst die Steuervorrichtung einen Konverter, der dazu eingerichtet ist, über die erste Schnittstelle erste Steuerungsdaten zu empfangen, die für das wenigstens eine Vorschaltgerät bestimmt sind, und aus den ersten Steuerungsdaten gemäß einer Parametrierung zweite Steuerungsdaten zu erzeugen, die sich von den ersten Steuerungsdaten unterscheiden. Hierbei erfolgt nicht unbedingt eine Veränderung des Datenformats. Dieses kann beibehalten werden, so dass sich die ersten und zweiten Steuerungsdaten nur inhaltlich unterscheiden. Unter den Begriff der empfangenen ersten Steuerungsdaten sind auch Befehle zu subsumieren, die sodann von der Steuervorrichtung umgesetzt werden und deren Ausführung an den oder die an die Steuervorrichtung angeschlossenen Vorschaltgeräte der Leuchtmittel geleitet werden.

Die Steuervorrichtung tritt hierbei bezogen auf die Beleuchtungssteuerung wie ein Vorschaltgerät auf, d.h. sie kann die gleichen Steuerdaten empfangen wie ein solches Vorschaltgerät und kann sich optional, wie noch erläutert wird, auch aktiv gegenüber der Beleuchtungssteuerung als Vorschaltgerät zu erkennen geben. Gegenüber dem eigentlichen Vorschaltgerät tritt die Steuervorrichtung wie eine Beleuchtungssteuerung auf, d.h. weder Beleuchtungssteuerung noch Vorschaltgerät nehmen die Existenz der Steuervorrichtung wahr oder müssen an diese angepasst werden.

Erfindungsgemäß kann es sein, dass der Konverter beispielsweise bei Empfang bestimmter erster Steuerungsdaten oder innerhalb bestimmter Zeitintervalle aus den ersten Steuerungsdaten identische zweite Steuerungsdaten erzeugt. Ein solches "Durchreichen" der Steuerungsdaten geschieht aber nicht im Allgemeinen, sondern wenn überhaupt nur fall- bzw. zeitweise. Im Allgemeinen verändert bzw. manipuliert der Konverter die Daten, wobei diese Veränderung durch die Parametrierung des Konverters vorgegeben ist. Die Parametrierung kann hierbei die Einstellung verschiedenster Parameter umfassen. Beispielsweise kann hierüber eingestellt werden, dass ein bestimmter Dimmwert, der über den Daten-Bus empfangen wird, in einen (niedrigeren oder höheren) Dimmwert übersetzt wird, der einer anderen (höheren oder niedrigeren) Effizienz eines Leuchtmittels entspricht. Es versteht sich, dass der Konverter wenigstens teilweise softwaremäßig realisiert sein kann, insbesondere als Programm, das von einem Prozessor der Steuereinheit verarbeitet wird.

Die Steuervorrichtung kann hierbei nach Art eines Routers fungieren, der die Steuerdaten im Wesentlichen quantitativ verändert, z.B. so dass die Helligkeit des Leuchtmittels mit einem Gewichtungsfaktor modifiziert wird. Ansonsten werden die Steuerdaten hier im Wesentlichen an das Vorschaltgerät weitergeleitet.

Alternativ kann die Steuervorrichtung auch als eine Art Gateway fungieren. Sie kann in diesem Fall mittels der ersten Steuerungsdaten einen Befehl erhalten und diesen umsetzen. Bei einem solchen Befehl kann es sich beispielsweise um das Abspielen einer Lichtszenerie handeln, die mittels eines oder mehrerer Leuchtmittel umgesetzt wird. Insofern erhält die Steuervorrichtung nur diesen Befehl und arbeitet dann ein hiermit verbundenes Programm, etwa eine Lichtszenerie, aufgrund der intern gespeicherten Daten ab. Der eine Befehl impliziert hierbei also Steuerdaten für die einzelnen Leuchtmittel. Die ersten Steuerdaten (ein Befehl) unterscheiden sich in diesem Fall auch qualitativ von den zweiten Steuerdaten (Steuersequenzen für mehrere Leuchtmittel). Die ersten Steuerungsdaten können somit hinsichtlich ihres Formats oder ihres Inhalts so beschaffen sein, dass das Vorschaltgerät diese nicht unmittelbar verarbeiten könnte, wenn die erfindungsgemäße Steuervorrichtung fehlen würde. Sie sind aber auch in diesem Fall im Sinne der Erfindung "für das Vorschaltgerät bestimmt", als sie eine bestimmte Ansteuerung desselben implizieren. Auch in diesem Fall kann die Steuervorrichtung die seitens der Beleuchtungssteuerung (implizit) gesendeten Steuerdaten in veränderter Form an die Leuchtmittel senden. D.h. wenn der empfangene Befehl für die Lichtszenerie beispielsweise für ein Leuchtmittel einen Betriebsstrom von 100% impliziert, kann die Steuervorrichtung gemäß der Parametrierung eine bessere Effizienz dieses Leuchtmittels dadurch berücksichtigen, dass es nur mit 80% angesteuert wird.

Erfindungsgemäß ist die Parametrierung gegenüber einem Reset seitens der Beleuchtungssteuerung geschützt. Der Begriff "Reset" bezeichnet hierbei ein Zurücksetzen auf Standardwerte. Wird also durch die Beleuchtungssteuerung ein Reset-Signal über den Daten-Bus gesendet bzw. wird an der Beleuchtungssteuerung selbst ein Reset durchgeführt, so ist die Parametrierung des Konverters hiervon nicht betroffen. Dies kann in verschiedener Weise erreicht werden, beispielsweise dadurch dass die Parametrierung einmalig in einem ROM, einem Flash-Speicher, einem EEPROM oder dergleichen abgelegt wird und bei oder nach Inbetriebnahme nicht mehr verändert werden kann, dadurch dass ein Speicher, in dem die Parametrierung abgelegt ist, keinerlei Verbindung zum Datenbus aufweist, oder dadurch, dass ein über den Daten-Bus empfangenes Reset-Signal innerhalb der Steuervorrichtung nicht derart verarbeitet wird, dass die Parametrierung hierdurch zurückgesetzt wird.

Die Steuervorrichtung ist weiterhin dazu eingerichtet, die zweiten Steuerungsdaten über die zweite Schnittstelle an das wenigstens eine Vorschaltgerät zu senden. Das Vorschaltgerät erhält also im Allgemeinen manipulierte bzw. veränderte Steuerungsdaten, wobei die Parametrierung, gemäß der die Veränderung vorgenommen wird, bei einem Reset nicht verloren geht.

Die Grundidee der Erfindung ist hierbei, dass einer Parametrierung, die eine optimale Steuerung des Leuchtmittels gewährleistet, im Idealfall nur einmal vorgenommen werden muss und hierbei vor einer unerwünschten bzw. unbeabsichtigten Löschung durch die übergeordnete Beleuchtungssteuerung geschützt ist. Die erfindungsgemäße Steuervorrichtung agiert hierbei gewissermaßen als Interface zwischen dem Datenbus und dem elektronischen Vorschaltgerät. Es kann somit eine optimale Steuerung des Leuchtmittels gewährleistet werden, ohne dass die Beleuchtungssteuerung hierfür speziell angepasste Steuerbefehle senden müsste oder dass eine spezielle Parametrierung des elektronischen Vorschaltgeräts notwendig wäre. Die Steuervorrichtung empfängt über den Datenbus von der Beleuchtungssteuerung "unangepasste" Steuerungsdaten, passt diese an die speziellen Gegebenheiten des Leuchtmittels an und stellt diese angepassten Daten dem Vorschaltgerät des Leuchtmittels zur Verfügung. Es versteht sich, dass die Parametrierung an geänderte Umstände angepasst werden kann, beispielsweise wenn das oder die an die Steuervorrichtung angeschlossenen Leuchtmittel gegen andere ausgetauscht werden.

Physisch kann die Steuervorrichtung unmittelbar an dem Vorschaltgerät, unmittelbar in der Nähe der Beleuchtungssteuerung oder auch deutlich von beiden beabstandet angeordnet sein.

Vorteilhaft verfügt die Steuervorrichtung über eine eigene Spannungsversorgung, die beispielsweise im Falle eines DALI-Systems von dessen Spannungsversorgung unabhängig ist.

Wie bereits erwähnt, wird im Allgemeinen keine Veränderungen des Datenformats vorgenommen. Besonders vorteilhaft lässt sich die Erfindung anwenden, wenn die Steuervorrichtung innerhalb eines DALI-Systems eingesetzt wird. Bevorzugterweise ist daher die erste Schnittstelle zum Anschluss an einen DALI-Bus vorgesehen und der Konverter ist dazu eingerichtet, die ersten Steuerungsdaten im DALI-Format zu empfangen und die zweiten Steuerungsdaten im DALI-Format zu erzeugen.

Weiterhin ist es zweckmäßig, wenn der Konverter dazu eingerichtet ist, Steuerungsdaten für ein Vorschaltgerät einer LED-Leuchte zu erzeugen. In diesem Fall ist die Steuervorrichtung somit für ein elektronisches Vorschaltgerät, also einen Treiber, einer LED-Leuchte vorgesehen. Eine solche LED-Leuchte kann eine oder - typischerweise - eine Mehrzahl von LEDs umfassen.

Vorteilhaft ist durch die Parametrierung eine zeitabhängige Abnahme der Effektivität des Leuchtmittels kompensierbar. Wie bereits erwähnt, unterliegt beispielsweise eine LED einer zeitlichen Effektivitätsabnahme (Degradation), so dass am Ende der Nutzungsdauer der LED bei gleicher Stromaufnahme möglicherweise nur noch ca. 70-80 % der Leuchtleistung einer neuen LED vorhanden sind. Dies kann durch eine entsprechende Parametrierung kompensiert werden, bei der im Anfang der Lebensdauer mit beispielsweise 70% des Betriebsstroms gearbeitet wird, der jedoch 100% des gewünschten Lichtstroms durch das Leuchtmittel, beispielsweise durch die LED bereitstellt. Während Benutzung wird dieser Betriebsstromwert nach und nach bis zum Ende der Nutzungsdauer auf 100% gesteigert wird. Anders gesagt, ein seitens der Beleuchtungssteuerung vorgegebener Wert wird mit einem Gewichtungsfaktor modifiziert, der während der Lebensdauer der LED nach und nach gesteigert wird. Die Entwicklung des Gewichtungsfaktors über die Zeit kann hierbei vorab gemäß einer empirisch ermittelten oder werksseitig vorgegebenen Kennlinie als Parameter hinterlegt sein. Durch die Steigerung des Gewichtungsfaktors kann einerseits während der gesamten Nutzungsdauer eine ausreichende Beleuchtung gewährleistet werden, andererseits wird keine Energie verschwendet, etwa dadurch, dass in Vorwegnahme der späteren geringeren Effizienz die LED von vornherein mit einem höheren Strom betrieben würde.

In einer weiteren Ausgestaltung ist durch die Parametrierung eine Typabhängigkeit der Effektivität des Leuchtmittels kompensierbar. Hierdurch kann dem Umstand Rechnung getragen werden, dass z.B. LEDs unterschiedlicher Hersteller oder unterschiedlicher Produktgenerationen unterschiedlich effizient arbeiten. Insbesondere ist typischerweise eine Produktgeneration effektiver als die vorherige. Um zu vermeiden, dass effizienter arbeitende Leuchtmittel mit einem zu hohen Betriebsstrom betrieben werden, kann ein seitens der Beleuchtungssteuerung vorgegebener Wert wird mit einem Gewichtungsfaktor modifiziert werden, der vom Typ (Hersteller und/oder Produktgeneration) des Leuchtmittels abhängt. Dieser Gewichtungsfaktor kann optional, wie oben beschrieben, auch noch zeitabhängig sein, um einen Alterungsprozess des Leuchtmittels zu berücksichtigen.

Die beiden oben dargestellten Ausführungsformen der Erfindung lassen sich darunter subsumieren, dass ein seitens der Beleuchtungssteuerung durch die ersten Steuerungsdaten vorgegebener Betriebsstrom des wenigstens einen Leuchtmittels durch die Parametrierung mit einem Gewichtungsfaktor skaliert bzw. modifiziert wird und in Form der zweiten Steuerungsdaten an das Vorschaltgerät gesendet wird. Dieser Gewichtungsfaktor kann zeitabhängig oder zeitunabhängig sein. Außer zur Effizienzkompensation kann ein solcher Gewichtungsfaktor auch beispielsweise aufgrund von individueller Kundenwünsche oder zur Begrenzung der Erwärmung des Leuchtmittels eingeführt werden.

Gemäß einer Ausgestaltung ist die Steuervorrichtung zum Senden der zweiten Steuerungsdaten an eine Mehrzahl von elektronischen Vorschaltgeräten und damit an eine Mehrzahl von Leuchtmitteln eingerichtet. Das Senden kann hierbei über eine einzelne zweite Schnittstelle, die beispielsweise an einen Daten-Bus angeschlossen ist, an den auch die elektronischen Vorschaltgeräte angeschlossen sind, oder über eine Mehrzahl von zweiten Schnittstellen erfolgen. Hierbei kann die Steuervorrichtung eingerichtet sein, die elektronischen Vorschaltgeräte einzeln zu adressieren (Unicast oder Multicast), oder die Steuerungsdaten können unspezifisch, also im Broadcast gesendet werden. Letzteres bietet sich insbesondere dann an, wenn eine Vielzahl von grundsätzlich gleich gearteten Leuchtmitteln mit entsprechenden Vorschaltgeräten gesteuert werden soll. In jedem Fall kann hier mit einer einzelnen Steuervorrichtung eine adäquate Steuerung einer Mehrzahl von Leuchtmitteln erfolgen. Auf eine Parametrierung der einzelnen Leuchtmittel bei Inbetriebnahme kann verzichtet werden.

Gemäß einer weiteren Ausgestaltung der Erfindung ist der Konverter dazu eingerichtet, für unterschiedliche Vorschaltgeräte unterschiedliche zweite Steuerungsdaten zu erzeugen. In diesem Fall können mittels eines Konverters einzelne Leuchtmittel in unterschiedlicher Weise angesteuert werden. Dies kann beispielsweise dazu dienen, einen unterschiedlichen Typ, ein unterschiedliches Alter (verbunden mit unterschiedlicher Effizienz) oder eine unabhängig hiervon gewünschte unterschiedliche Betriebsart der einzelnen Leuchtmittels zu berücksichtigen.

Hierbei ist der Konverter bevorzugt dazu eingerichtet, die unterschiedlichen zweiten Steuerungsdaten aus unterschiedlichen ersten Steuerungsdaten zu erzeugen, die für unterschiedliche Vorschaltgeräte bestimmt sind. In diesem Fall verarbeitet der Konverter also erste Steuerungsdaten, die von vornherein für unterschiedliche Vorschaltgeräte bestimmt sind, wandelt diese jeweils gemäß seiner Parametrierung um, wonach die so erzeugten zweiten Steuerungsdaten an die entsprechenden Vorschaltgeräte gesendet werden. Im Extremfall könnte hier eine einzige Steuervorrichtung sämtlichen Leuchtmitteln eines Beleuchtungssystems zugeordnet sein.

Gemäß einer weiteren vorteilhaften Ausführungsform ist der Konverter für den optimalen Betrieb einer Mischlichtquelle ausgelegt. Als solche Mischlichtquelle kommt beispielsweise eine so genannte "tunable-white"-Leuchte infrage, bei der unterschiedlich warme oder kalte Lichttöne durch Mischen des Lichts verschiedener LEDs erzeugt werden. Selbstverständlich kommen auch z.B. RGB-Leuchten infrage. Während hier typischerweise von der Beleuchtungssteuerung ein richtiges Mischungsverhältnis der beiden Lichtarten vorgegeben wird, ist es oftmals so, dass die Beleuchtungssteuerung Lichtströme vorgibt, die einer zu hohen Gesamtlichtstärke entsprechen. Dies wiederum führt zu einem unnötigen Energieverbrauch und ggf. zu einer unerwünschten Erwärmung, die zu einem geringeren Lichtstrom und einer geringeren Lebensdauer führen kann. In diesem Fall kann durch die erfindungsgemäße Steuervorrichtung eine Skalierung der Lichtströme vorgenommen werden. Vorteilhaft sind daher durch die Parametrierung zweite Steuerungsdaten für die Leuchtmittel einer Mischlichtquelle derart erzeugbar, dass unabhängig vom Mischverhältnis eine konstante Gesamthelligkeit gegeben ist. Der Begriff "konstant" ist hierbei selbstverständlich nicht streng auszulegen, tatsächlich wird sich die Gesamthelligkeit innerhalb eines technisch vernünftig realisierbaren, engen Bereichs bewegen. D.h., wenn sich bei verschiedenen Mischungsverhältnis Helligkeitsschwankungen im Bereich von ca. 5%, ggf. auch 10%, ergeben, ist dies in diesem Zusammenhang noch als konstante Helligkeit aufzufassen. Selbstverständlich ist es auch denkbar, dass das Mischungsverhältnis der verschiedenen Lichtanteile über die zweiten Steuerungsdaten manipuliert wird.

Moderne Steuerungssysteme wie DALI zeichnen sich dadurch aus, dass ein bidirektionale Datenfluss möglich ist, d.h. dass die einzelnen Aktoren über ihre Schnittstellen und den Daten-Bus auch Rückmeldungen an die Beleuchtungssteuerung geben können. Um diese Möglichkeit zu erhalten, ist die Steuervorrichtung gemäß einer bevorzugten Ausgestaltung der Erfindung dazu eingerichtet, von dem wenigstens einen Vorschaltgerät gesendete Rückmeldedaten über die zweite Schnittstelle zu empfangen und über die erste Schnittstelle an den Daten-Bus zu senden. Falls seitens des Vorschaltgerät hierbei ein anderes Datenformat verwendet wird als im Daten-Bus, kann hierbei eine Umwandlung des Datenformats erfolgen. Eine inhaltliche Manipulation der so übermittelten Daten ist nicht vorgesehen. Bei dieser Ausgestaltung ist selbstverständlich auch möglich, dass die Steuervorrichtung über die zweite Schnittstelle Anfragen an das Vorschaltgerät sendet - oder eine über die erste Schnittstelle empfangene Anfrage weiterleitet - und als Reaktion hierauf die Rückmeldedaten empfängt.

Wie bereits eingangs erläutert, kann die Parametrierung des Konverters ggf. in einem ROM, Flash-Speicher o.ä. einmalig vorgegeben sein. Vorteilhaft ist es allerdings, wenn eine nachträgliche Vornahme bzw. Veränderung der Parametrierung möglich ist. Hierfür kann die Steuervorrichtung eine zusätzliche, dritte Schnittstelle aufweisen, wobei der Speicher für die Parameter somit vollständig vom Daten-Bus getrennt sein kann. In einer alternativen, bevorzugten Ausführung ist die Parametrierung des Konverters durch Dateneingabe über die erste oder zweite Schnittstelle vornehmbar. D.h., während ein Reset-Signal, das über die erste Schnittstelle eingeht, die Parametrierung nicht beeinflusst, kann beispielsweise mittels proprietärer Befehle, die über die erste Schnittstelle oder aber die zweite Schnittstelle eingespeist werden, die Parametrierung geändert bzw. anfänglich eingestellt werden. Im Falle eines DALI-Systems kann hier beispielsweise mittels proprietärer Befehle gearbeitet werden, die über eine Hersteller-Identifikation (Vendor-ID) verschlüsselt sind. Bei Vorliegen der richtigen Identifikation kann mittels solcher Befehle die Parametrierung verändert werden.

Gemäß einer anderen Ausgestaltung ist die Steuervorrichtung dazu eingerichtet, Messdaten eines Sensors zu empfangen und die zweiten Steuerungsdaten in Abhängigkeit von den Messdaten zu erzeugen. Hierbei kann der Sensor Teil der Steuervorrichtung sein oder aber von dieser getrennt sein, wobei die Messdaten über eine der Schnittstellen in die Steuervorrichtung eingespeist werden können. Durch die Messdaten des Sensors kann z.B. die tatsächliche Helligkeit einer LED-Leuchte gemessen werden, und die Steuervorrichtung kann bei einer Abweichung von der erwarteten Helligkeit die zweiten Steuerungsdaten so anpassen, dass die Abweichung ausgeglichen wird. Denkbar ist beispielsweise auch der Einsatz von Temperatursensoren, mittels derer Referenztemperaturen für Schalt- oder Regelprozesse ermittelt werden können. In jedem Fall besteht die Möglichkeit, dass die Messdaten des Sensors über die erste Schnittstelle in den Daten-Bus eingespeist werden und somit auch anderen hieran angeschlossenen Komponenten zur Verfügung stehen.

Durch die Erfindung wird auch ein Steuerverfahren für wenigstens ein Leuchtmittel zur Verfügung gestellt, wobei eine Beleuchtungssteuerung erste Steuerungsdaten, die für wenigstens ein elektronisches Vorschaltgerät wenigstens eines Leuchtmittels bestimmt sind, über einen Daten-Bus sendet, ein Konverter die ersten Steuerungsdaten empfängt und aus den ersten Steuerungsdaten gemäß einer Parametrierung zweite Steuerungsdaten erzeugt, die sich von den ersten Steuerungsdaten unterscheiden, wobei die Parametrierung gegenüber einem Reset seitens der Beleuchtungssteuerung geschützt ist, und die zweiten Steuerungsdaten an das wenigstens eine Vorschaltgerät gesendet werden.

Bevorzugte Ausgestaltung des Steuerverfahrens entsprechend den oben dargestellten bevorzugten Ausgestaltung der Steuervorrichtung.

Weitere Vorteile und Ausgestaltungen der Erfindung werden nachfolgend anhand von Ausführungsbeispielen mit Bezug auf die Figuren erläutert. Hierbei zeigt:
- **Fig. 1:**: eine schematische Darstellung eines Beleuchtungssystems mit einer ersten Ausführungsform einer erfindungsgemäßen Steuervorrichtung,
- **Fig. 2:**: eine schematische Darstellung eines weiteren Beleuchtungssystems mit einer zweiten Ausführungsform einer erfindungsgemäßen Steuervorrichtung,
- **Fig. 3:**: ein Diagramm, das die zeitliche Entwicklung eines Gewichtungsfaktors darstellt, und
- **Fig. 4:**: ein Diagramm, das Helligkeiten einer Mischlichtquelle darstellt.

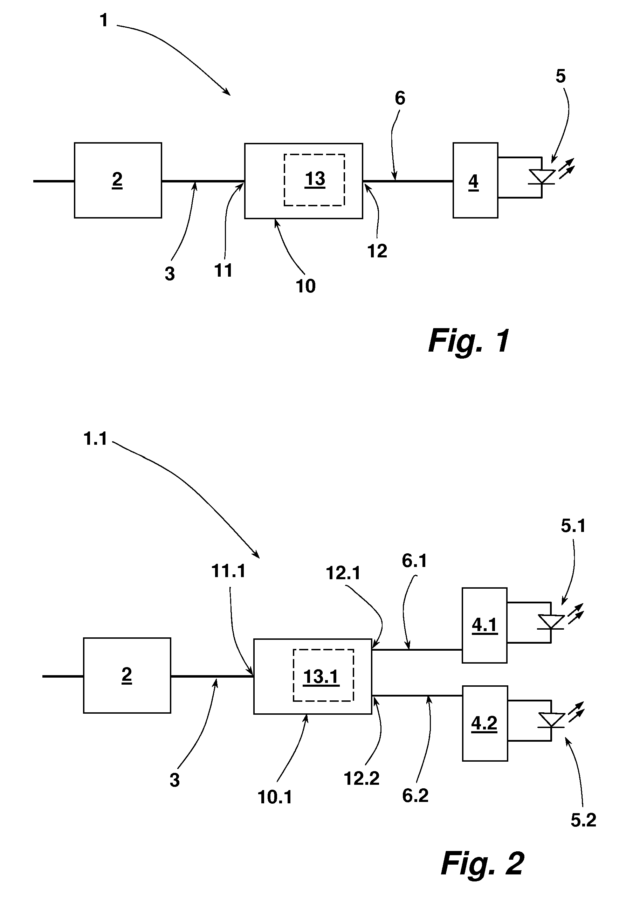

Bei dem in Figur 1 gezeigten Beleuchtungssystem 1 geht von einer zentralen Beleuchtungssteuerung 2 ein DALI-Bus 3 aus, über den die Beleuchtungssteuerung 2 mit verschiedenen Aktoren verbunden werden kann. Im vorliegenden Fall soll ein elektronisches Vorschaltgerät (EVG) 4 einer LED 5 gesteuert werden. Die LED 5 unterliegt allerdings während ihrer Lebensdauer von ca. 50.000 Stunden einer altersbedingten Effektivitätsabnahme. Während die Beleuchtungssteuerung 2 für eine bestimmte Helligkeit immer einen bestimmten, gleich bleibenden Wert für die Stromaufnahme vorgibt, soll im vorliegenden Fall das EVG 4 mit einem Wert angesteuert werden, der der zeitlichen Effektivitätsabnahme Rechnung trägt. Zu diesem Zweck ist zwischen den DALI-Bus 3 und das EVG 4 eine Steuervorrichtung 10 geschaltet, die die entsprechende Datenumwandlung vornimmt. Die Steuervorrichtung 10 ist über eine erste Schnittstelle 11 mit dem DALI-Bus 3 verbunden, über den für das EVG 4 bestimmte erste Steuerungsdaten gesendet werden. Über eine zweite Schnittstelle 12 sowie einen sekundären DALI-Bus 6 ist die Steuervorrichtung 10 mit dem EVG 4 verbunden. Des Weiteren verfügt die Steuervorrichtung 10 über eine eigene, unabhängige Spannungsversorgung (nicht dargestellt).

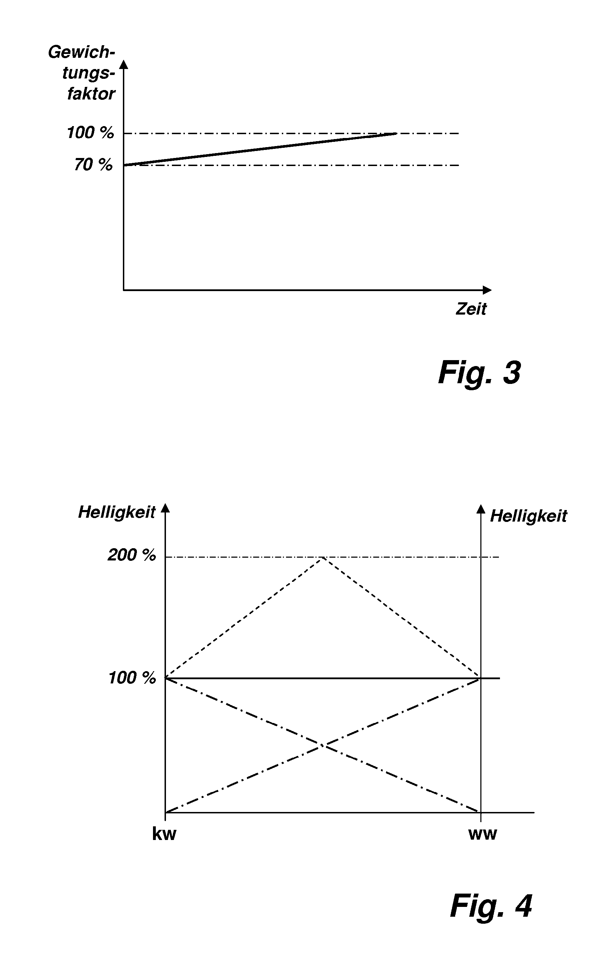

Die über den DALI-Bus 3 empfangenen ersten Steuerungsdaten werden von der Steuervorrichtung 10 als für das EVG 4 bestimmt erkannt und ein Konverter 13 der Steuervorrichtung 10 erzeugt hieraus zweite Steuerungsdaten, wobei die Stromaufnahme der LED 5 derart gesteuert wird, dass einer altersbedingten Verschlechterung der Effizienz Rechnung getragen wird. Im vorliegenden Fall ist der Konverter 13 als Computerprogramm realisiert, dass auf einem Prozessor (nicht dargestellt) der Steuervorrichtung 10 läuft. Um die Umwandlung der Steuerungsdaten richtig durchführen zu können, ist die Steuervorrichtung 10 mit einer Kennlinie der LED 5 parametriert, aus der sich ein zeitabhängiger Gewichtungsfaktor zur Steuerung der Stromaufnahme gibt. Die entsprechende Kennlinie ist im Diagramm in Figur 3 dargestellt. Vorliegend ist vorgesehen, dass der Gewichtungsfaktor, dargestellt durch die durchgezogene Linie, linear von 70% auf 100% erhöht wird. Die so modifizierten zweiten Steuerungsdaten werden über die zweite Schnittstelle 12 und den sekundären DALI-Bus 6 an das EVG 4 gesendet.

Die entsprechenden Parameter sind hierbei in einem nicht-flüchtigen Speicher der Steuervorrichtung 10 abgelegt. Durch proprietäre Befehle, die über den DALI-Bus 3 gesendet werden, lässt sich die Parametrierung der Steuervorrichtung 10 verändern. Die Befehle sind hierbei mit einer Vendor-ID verschlüsselt, die der Steuervorrichtung bekannt ist. Wird allerdings über den DALI-Bus 3 einen Reset-Signal gesendet und von der Steuervorrichtung 10 empfangen, so führt dies intern nicht zu einem Zurücksetzen der Parameter. Grundsätzlich muss also bei Inbetriebnahme der LED 5 lediglich einmal die richtige Parametrierung vorgenommen werden, wonach innerhalb deren Betriebsdauer keine erneute Parametrierung notwendig ist.

Die Steuervorrichtung 10 ist hierbei so konfiguriert, dass sie von der Beleuchtungssteuerung 2 als EVG 4 erkannt wird. Des Weiteren werden von der Beleuchtungssteuerung 2 an das EVG 4 gerichtete Anfragen über die erste und zweite Schnittstelle 11, 12 an das EVG 4 weitergeleitet und die entsprechenden Rückmeldedaten über die zweite und erste Schnittstelle 12, 11 an die Beleuchtungssteuerung 2 weitergegeben. Eine Modifizierung der entsprechenden Daten durch die Steuervorrichtung 10 erfolgt hierbei nicht.

In Figur 1 ist der Einfachheit halber nur ein EVG 4 dargestellt. Es versteht sich, dass das EVG 4 auch stellvertretend für mehrere oder auch eine Vielzahl von EVGs steht, die durch einen Broadcast von der Steuervorrichtung 10 angesprochen sind.

Figur 2 zeigt einen Beleuchtungssystem 1.1 mit einer weiteren Ausführungsform einer Steuervorrichtung 10.1, die über eine einzelne erste Schnittstelle 11.1 durch Verbindung mit dem DALI-Bus 3 verfügt, allerdings über zwei zweite Schnittstellen 12.1, 12.2. Ein erstes EVG 4.1 einer ersten LED 5.1 sowie einen zweites EVG 4.2 einer zweiten LED 5.2 sind über jeweils einen sekundären DALI-Bus 6.1, 6.2 mit einer der zweiten Schnittstellen 12.1, 12.2 verbunden. Die LEDs 5.1, 5.2 gehöre hierbei zu einer "tunable-white"-Leuchte, die weißes Licht unterschiedlich warmer Töne zur Verfügung stellt. Die Farbtemperatur (der Farbton) hängt hierbei vom Verhältnis der Helligkeiten der beiden LEDs 5.1, 5.2 ab. Durch die Beleuchtungssteuerung 2 werden erste Steuerungsdaten für die beiden EVGs 4. 1, 4.2 zur Verfügung gestellt, denen zufolge zunächst die Helligkeit der ersten Farbe konstant gehalten und die der zweiten linear erhöht wird, bis beide Farben mit gleichen Helligkeiten vorhanden sind. Diese entsprechen jeweils 100% der Helligkeit bei ungemischtem Licht, die einem Optimalwert entspricht. Dann wird die Helligkeit der ersten Farbe linear verringert, während die der zweiten konstant gehalten wird. Hieraus ergibt sich eine Helligkeitsentwicklung wie durch die strichpunktierte Linie in Figur 4 gezeigt. Man erhält hier eine veränderliche, teilweise unerwünscht hohe Gesamthelligkeit (bis zu 200% des Optimalwerts), verbunden mit einem hohen Energieverbrauch.

Aus diesem Grund werden in einem Konverter 13.1 der Steuervorrichtung 10.1 aus den ersten Steuerungsdaten zweite Steuerungsdaten erzeugt, gemäß derer die Gesamthelligkeit so gesteuert wird, dass diese unabhängig vom Mischungsverhältnis der Farbtöne (in etwa) konstant bleibt, wie in Figur 4 durch die durchgezogene Linie dargestellt. Es wird hierbei die Helligkeit der einen Farbe linear z.B. kaltweiss (kw) von 0 auf 100% erhöht (in Figur 4 durch die strichpunktierte Linie dargestellt), während die Helligkeit der anderen Farbe z.B. warmweiss (ww) gleichzeitig linear von 100% auf 0 verringert wird (in Figur 4 durch die ebenfalls strichpunktierte andere Linie dargestellt). Die entsprechenden zweiten Steuerungsdaten werden über die zweiten Schnittstellen 12.1, 12.2 an die EVGs 4. 1, 4.2 gesendet. Selbstverständlich könnten die Helligkeiten alternativ auch nicht-linear verändert werden, solange die Summe in etwa 100% des Optimalwerts ergibt.

Die weitere Ausgestaltung der Steuervorrichtung 10.1 unterscheidet sich nicht von der ersten Ausführungsbeispiels, weshalb diese nicht nochmals erläutert wird. Allerdings ist die Steuervorrichtung 10.1 vorliegend so konfiguriert, dass sie von der Beleuchtungssteuerung 2 als zwei Geräte, nämlich EVG 4. 1 und EVG 4. 2 erkannt wird.

Auch bezüglich des Ausführungsbeispiels der Figur 2 stehen die EVGs 4.1 und 4.2 sowohl für sich alleine als auch stellvertretend für mehrere, an den jeweiligen sekundären DALI-Bus angeschlossenen EVGs.

Die Erfindung ist anhand von konkreten Ausführungsbeispielen beschrieben worden. Für einen Fachmann ergeben sich zahlreiche weitere Möglichkeiten, die Erfindung im Rahmen der geltenden Ansprüche zu verwirklichen.

### Bezugszeichenliste

- 1, 1.1: Beleuchtungssystem
- 2: Beleuchtungssteuerung
- 3: DALI-Bus
- 4,4.1, 4.2: EVG
- 5,5.1, 5.2: LED
- 6, 6.1, 6.2: sekundärer DALI-Bus
- 10, 10.1: Steuervorrichtung
- 11, 11.1: erste Schnittstelle
- 12, 12.1, 12.2: zweite Schnittstelle
- 13, 13.1: Konverter

## Patentansprüche

1. Steuervorrichtung (10, 10.1) für wenigstens ein Leuchtmittel (5, 5.1, 5.2) mit
- wenigstens einer ersten Schnittstelle (11, 11.1) zum Anschluss an einen Daten-Bus (3) einer Beleuchtungssteuerung (2),
- wenigstens einer zweiten Schnittstelle (12, 12.1, 12.2) zum Anschluss an wenigstens ein elektronisches Vorschaltgerät (4, 4.1, 4.2) des wenigstens einen Leuchtmittels (5, 5.1, 5.2), sowie
- einem Konverter (13, 13.1), der dazu eingerichtet ist, über die erste Schnittstelle (11, 11.1) erste Steuerungsdaten zu empfangen, die für das wenigstens eine Vorschaltgerät (4, 4.1, 4.2) bestimmt sind, und aus den ersten Steuerungsdaten gemäß einer Parametrierung zweite Steuerungsdaten zu erzeugen, die sich von den ersten Steuerungsdaten unterscheiden, wobei die Parametrierung gegenüber einem Reset seitens der Beleuchtungssteuerung (2) geschützt ist,
wobei die Steuervorrichtung (10, 10.1) dazu eingerichtet ist, die zweiten Steuerungsdaten über die zweite Schnittstelle (12, 12.1, 12.2) an das wenigstens eine Vorschaltgerät (4, 4.1, 4.2) zu senden.

2. Steuervorrichtung nach Anspruch 1, **dadurch gekennzeichnet, dass** die erste Schnittstelle (11, 11.1) zum Anschluss an einen DALI-Bus vorgesehen ist und der Konverter (13, 13.1) dazu eingerichtet ist, die ersten Steuerungsdaten im DALI-Format zu empfangen und die zweiten Steuerungsdaten im DALI-Format zu erzeugen.

3. Steuervorrichtung nach Anspruch 1 oder 2, **dadurch gekennzeichnet, dass** der Konverter (13, 13.1) dazu eingerichtet ist, Steuerungsdaten für ein Vorschaltgerät (4, 4.1, 4.2) einer LED-Leuchte (5, 5.1, 5.2) zu erzeugen.

4. Steuervorrichtung nach einem oder mehreren der vorangegangen Ansprüche, **dadurch gekennzeichnet, dass** durch die Parametrierung eine zeitabhängige Abnahme der Effektivität des Leuchtmittels (5) kompensiert ist.

5. Steuervorrichtung nach einem oder mehreren der vorangegangen Ansprüche, **dadurch gekennzeichnet, dass** durch die Parametrierung eine Typabhängigkeit der Effektivität des Leuchtmittels (5) kompensiert ist.

6. Steuervorrichtung nach einem oder mehreren der vorangegangen Ansprüche, **dadurch gekennzeichnet, dass** diese zum Senden der zweiten Steuerungsdaten an eine Mehrzahl von elektronischen Vorschaltgeräten (4.1, 4.2) eingerichtet ist.

7. Steuervorrichtung nach einem oder mehreren der vorangegangen Ansprüche, **dadurch gekennzeichnet, dass** der Konverter (13.1) dazu eingerichtet ist, für unterschiedliche Vorschaltgeräte (4.1, 4.2) unterschiedliche zweite Steuerungsdaten zu erzeugen.

8. Steuervorrichtung nach Anspruch 7, **dadurch gekennzeichnet, dass** der Konverter (13.1) dazu eingerichtet ist, die unterschiedlichen zweiten Steuerungsdaten aus unterschiedlichen ersten Steuerungsdaten zu erzeugen, die für unterschiedliche Vorschaltgeräte (4.1, 4.2) bestimmt sind.

9. Steuervorrichtung nach Anspruch 8, **dadurch gekennzeichnet, dass** durch die Parametrierung zweite Steuerungsdaten für die Leuchtmittel (5.1, 5.2) einer Mischlichtquelle derart erzeugbar sind, dass unabhängig vom Mischverhältnis eine konstante Gesamthelligkeit gegeben ist.

10. Steuervorrichtung nach einem oder mehreren der vorangegangen Ansprüche, **dadurch gekennzeichnet, dass** die Steuervorrichtung dazu eingerichtet ist, von dem wenigstens einen Vorschaltgerät (4, 4.1, 4.2) gesendete Rückmeldedaten über die zweite Schnittstelle (12, 12.1, 12.2) zu empfangen und über die erste Schnittstelle (11, 11.1) an den Daten-Bus (3) zu senden.

11. Steuervorrichtung nach einem oder mehreren der vorangegangen Ansprüche, **dadurch gekennzeichnet, dass** die Parametrierung des Konverters (13, 13.1) durch Dateneingabe über die erste (11, 11.1) oder zweite Schnittstelle (12, 12.1, 12.2) vornehmbar ist.

12. Steuervorrichtung nach einem oder mehreren der vorangegangen Ansprüche, **dadurch gekennzeichnet, dass** sie dazu eingerichtet ist, Messdaten eines Sensors zu empfangen und die zweiten Steuerungsdaten in Abhängigkeit von den Messdaten zu erzeugen.

13. Steuerverfahren für wenigstens ein Leuchtmittel, wobei
- eine Beleuchtungssteuerung (2) erste Steuerungsdaten, die für wenigstens ein elektronisches Vorschaltgerät (4, 4.1, 4.2) wenigstens eines Leuchtmittels (5, 5.1, 5.2) bestimmt sind, über einen Daten-Bus (3) sendet,
- ein Konverter (13, 13.1) die ersten Steuerungsdaten empfängt und aus den ersten Steuerungsdaten gemäß einer Parametrierung zweite Steuerungsdaten erzeugt, die sich von den ersten Steuerungsdaten unterscheiden, wobei die Parametrierung gegenüber einem Reset seitens der Beleuchtungssteuerung geschützt ist, und
- die zweiten Steuerungsdaten an das wenigstens eine Vorschaltgerät (4, 4.1, 4.2) gesendet werden.
